# EUROPEAN PATENT SPECIFICATION

(11) **EP 3 600 264 B1**
(45) Date of publication and mention of the grant of the patent: **04.12.2024**
(21) Application number: 18772394.5
(22) Date of filing: 23.03.2018
(51) Int. Cl.: A61N 1/04, A61K 9/70, A61N 1/30, A61N 1/32, A61M 5/00, A61M 37/00

(54) **MICROCELL DELIVERY SYSTEMS INCLUDING CHARGED PHASES AND CONTROLLERS FOR MICROCELL DELIVERY SYSTEMS**
MIKROZELLENEINFÜHRUNGSSYSTEME MIT GELADENEN PHASEN UND STEUERGERÄTE FÜR MIKROZELLENEINFÜHRUNGSSYSTEME
SYSTÈME D'ADMINISTRATION DE MICROCELLULES CONTENANT DES PHASES CHARGÉES ET DES DISPOSITIFS RÉGULATEURS DE SYSTÈMES D'ADMINISTRATION DE MICROCELLULES

(30) Priority: 24.03.2017 US 201762476176 P
(43) Date of publication of application: 05.02.2020
(73) Proprietor: E Ink Corporation, Billerica, MA 01821 (US)
(72) Inventor: LIU, Lei, Fremont California 94538 (US); ZANG, HongMei, Fremont California 94538 (US)
(74) Representative: Kilburn & Strode LLP
(86) International application number: PCT/US2018/023928
(87) International publication number: WO 2018/175834

(56) References cited:
- WO-A1-2018/175825
- US-A- 4 734 090
- US-A1- 2005 273 046
- US-A1- 2006 009 731
- US-A1- 2008 020 007
- US-A1- 2012 176 663
- US-A1- 2012 176 663
- US-A1- 2012 232 464

## Description

### BACKGROUND

Transdermal delivery of pharmaceutical agents has proven effective for drugs that are able to move across the skin barrier. For example, small amounts of nicotine can be delivered over extended periods with transdermal patches that suspend the nicotine in an ethylene vinyl acetate (EVA) copolymer. See, e.g., Nicoderm-CQ^{®} by GlaxoSmithKline (Brentford, UK). However, it is not possible to actively control the rate of administration. Rather, a series of different concentrations of nicotine suspended in EVA are provided to a user with instructions to use different patches on different days depending upon the treatment program or the level of craving.

It is recognized that the constant dosage delivered by a passive matrix of active agents may not be optimal for treating all conditions, however. For example, with respect to smoking cessation, it is recognized that the average smoker has cyclical cravings corresponding with normal daily activity, such as a waking, meals, etc. Accordingly, for some patients it is better to use a "dual therapy" including a transdermal patch and a fast acting delivery method, such as nicotine gum. See, Ebbert et al., Drugs 2010, 70(6), 643-650. Such dosing helps to wean the body from the dependency while also being responsive to the cyclical peak cravings. Other transdermally-delivered actives, such as insulin, also require "boosters" to overcome daily metabolic swings, e.g., following meals.

New "smart" transdermal patches that provide some ability to control dosing in real time, are currently being evaluated. For instance, Chrono Therapeutics (Hayward, CA) is currently testing a micropump-enabled smart transdermal patch for delivering nicotine. Chrono's device includes a "crave" button that allows users to receive a "booster" when cravings strike. Nonetheless, the Chrono device is larger than a typical transdermal patch and, thus, is visible through clothing as a sizeable bump. It also requires replacement cartridges and charging to maintain function. It is clear that there remains a need for a simple (and inexpensive) delivery system that provides for real-time modification of dosing.

US 2008020007 A1 describes a film structure which comprises a plurality of microcups and the microcups are filled with a liquid composition and top-sealed with a sealing layer which is hardened in situ. The liquid composition may be a display fluid or a pharmaceutical composition. The application mentions a liquid crystal display, a display device and a transdermal delivery system.
US 2012176663 A1 relates to an electrophoretic display comprising: (a) microcups comprising partition walls and top-openings; (b) an organic-based electrophoretic fluid filled in the microcups, wherein said fluid comprises charged pigment particles dispersed in a solvent; and (c) a top-sealing layer formed from a sealing composition to enclose the electrophoretic fluid within the microcups. The sealing composition comprises: (i) a water soluble polymer, (ii) a water-based suspension, a water-based dispersion, a water-based emulsion, or a water-based latex, each comprising a polymer; and (iii) water.

### SUMMARY

This invention provides an active molecule delivery system and active molecule delivery system controller in accordance with the appended claims. The invention addresses this need by providing a low power transdermal delivery system whereby the active molecules can be released on demand. Additionally, as described below, the invention provides a system for delivering varying concentrations of active molecules from the same delivery system at different times, and for delivering multiple drugs at the same, or different, times from the same patch.

Thus, the present disclosure concerns an active molecule delivery system including a plurality of microcells. The microcells may be square, round, or polygonal, such as a honeycomb structure. Each microcell includes an opening that is spanned by a porous diffusion layer. The porous diffusion layer may be constructed from a variety of materials, such acrylate, methacrylate, polycarbonate, polyvinyl alcohol, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polybutylene, polypropylene, polyisobutylene, or polystyrene. Typically, each microcell has a volume greater than 100 nL, and the porous diffusion layer has an average pore size of between 1 nm and 100 nm.

The microcells can be filled with a variety of materials. In one embodiment, the microcells are filled with a mixture of an active molecule dispersed in a first charged phase and a second phase that is oppositely charged or uncharged and immiscible with the first phase. The system additionally includes a source of an electric field. In another embodiment, the microcells are filled with a mixture of an active molecule and charged particles, and the system includes a source of an electric field. The charged particles may comprise a charged core particle and a polymer layer surrounding the core particle. In some embodiments, the mixture further includes a plurality of charge control agents.

In embodiments where the mixture includes active molecules dispersed in a first charged phase as well as an immiscible second phase, various combinations of hydrophobic and hydrophilic liquids, charged polymers, salts, ions, and surfactants may be used to stabilize the active and achieve the desired control over movement of the phases. The first charged phase comprises a biocompatible non-polar liquid or an aqueous liquid. If the first phase is a biocompatible non-polar liquid, the liquid could be a vegetable, fruit, or nut oil. The biocompatible non-polar liquid may additionally include charged hydrophobic polymers, such as functionalized acrylates, for example N-methacryloyl-L-phenyl alanine (MAPA). In other embodiments, the first charged phase includes an aqueous liquid. The aqueous liquid may also include charged hydrophilic polymers, such as polyethylenimine (PEI), polyethylene glycol) (PEG), poly(vinyl alcohol) (PVA), poly(vinylpyrrolidone) (PVP) or copolymers of any of these polymers. In some embodiments, the first charged phase includes a phosphate buffer. Whether the first charged phase is hydrophilic or hydrophobic will depend in good part upon the nature of the active to be delivered. Accordingly, where the first charged phase, including the active, is hydrophilic, the second phase will be hydrophobic, and where the first charged phase, including the active, is hydrophobic the second phase will be hydrophilic. The second phase may include any of the components above, as needed to engineer the proper response for the first charged phase. In some embodiments, the second phase will be charged, however, in other embodiments, the second phase will not be charged.

In some embodiments, the source of an electric field is first and second electrodes, whereby the mixture of an active molecule and charged particles is sandwiched between the first and second electrodes. For example, the first electrode or the second electrode could be part of an active matrix of electrodes. This active matrix may allow the delivery rate of individual microcells to be controlled. In some embodiments, the first electrode or the second electrode is porous, for example a porous film coated with a conductor such as indium tin oxide (ITO). Alternatively, the first or the second electrode may include a matrix of nanotubes that are both conductive and porous to the active molecules to be delivered.

Typically, the active molecule is a pharmaceutical compound, however systems of the invention can be used to deliver hormones, nutraceuticals, proteins, nucleic acids, antibodies, or vaccines. Because the invention includes a plurality of microcells, it is possible to have different microcells within the same device containing different mixtures or similar mixtures having different concentrations. For example, a system may include first microcells containing a mixture of first active molecules and second microcells containing a mixture of second active molecules, or a system may include first microcells containing active molecules at a first concentration and second microcells containing the same active molecules at a second concentration. In other embodiments, the system may include first microcells containing a mixture of active molecules and second microcells containing an adjuvant. Other combinations of active molecules, agents, and concentrations will be evident to one of skill in the art.

The present disclosure also concerns a controller for controlling an active molecule delivery system. This controller includes an active molecule delivery system including a mixture of an active molecule and charged particles. The controller additionally includes a voltage source and a protective covering. The controller will typically include connectors configured to couple with the first and second electrodes of the active molecule delivery system. The controller may also include a switch configured to interrupt flow from the voltage source to the active molecule delivery system. The switch may be a mechanical switch or a digital switch, and the controller may include a processor for controlling the switch. In some embodiments, the controller will include a wireless receiver and a wireless transmitter, thereby allowing the controller to be interfaced with a device, such as a smart phone, docking station, smart watch, fitness tracker, etc.

### BRIEF DESCRIPTION OF THE DRAWINGS

FIG. 1A illustrates an embodiment of an active molecule delivery system including a plurality of microcells and charged particles that regulate movement of active molecules across a porous diffusion layer;
FIG. 1B illustrates an embodiment of an active molecule delivery system including a plurality of microcells wherein the microcells include a mixture comprising an active in a first charged phase and a second charged or uncharged phase that is immiscible with the first phase;
FIG. 2A illustrates an active molecule delivery system including a plurality of different types of actives and/or a plurality of concentrations of actives in the same delivery system. Because the microcells can be individually addressed with an active matrix, it is possible to provide varying actives on demand and also to produce complex dosing profiles;
FIG. 2B illustrates an active molecule delivery system wherein some of the active molecules are released when charged particles are moved away from the porous diffusion layer while other actives are prevented from being released when the charged particles are moved adjacent to the porous diffusion layer;
FIG. 3A shows a top view of an active molecule delivery system that includes first and second electrodes that sandwich an array of microcells that hold an active. The electrodes include connectors that allow the electrodes to be coupled to a power supply;
FIG. 3B shows a side view of an active molecule delivery system that includes first and second electrodes that sandwich an array of microcells that hold a mixture of an active and charged particles. A similar design can be implemented for use with mixtures that include an active molecule dispersed in a first charged phase and a second phase that is oppositely charged or uncharged and immiscible with the first phase;
FIG. 3C illustrates an embodiment of a controller for controlling an active molecule delivery system. The controller includes a power supply and connectors to interface with the electrodes of the active molecule delivery system. The controller additionally includes a protective covering;
FIG. 4 shows a method for making microcells using a roll-to-roll process;
FIGS. 5A and 5B detail the production of microcells for an active molecule delivery system using photolithographic exposure through a photomask of a conductor film coated with a thermoset precursor;
FIGS. 5C and 5D detail an alternate embodiment in which microcells for an active molecule delivery system are fabricated using photolithography. In FIGS. 5C and 5D a combination of top and bottom exposure is used, allowing the walls in one lateral direction to be cured by top photomask exposure, and the walls in another lateral direction to be cured bottom exposure through the opaque base conductor film. This process allows microcell walls to be prepared with varying porosity for use with lateral motion embodiments;
FIGS. 6A-6D illustrate the steps of filling and sealing an array of microcells to be used in an active molecule delivery system;
FIG. 7 illustrates an embodiment of an active molecule delivery system controller including a wireless receiver allowing a user to alter the rate of delivery with an application on a mobile phone or other wireless device;
FIG. 8 illustrates an embodiment of an active molecule delivery system controller for regulating an active matrix of electrodes via a wireless receiver, thereby allowing an external device to alter the type of active molecule that is being delivered;

### DESCRIPTION

The invention provides an active molecule delivery system whereby active molecules can be released on demand and/or a variety of different active molecules can be delivered from the same system and/or different concentrations of active molecules can be delivered from the same system. The invention is well-suited for delivering pharmaceuticals to patients transdermally, however the invention may be used to deliver active ingredients to animals, generally. For example, the invention can deliver tranquilizing agents to a horse during transport. The active delivery system includes a plurality of microcells, wherein the microcells are filled with a medium for delivering the active molecules. In some embodiments the medium includes active molecules dispersed in a first charged phase as well as a second phase that is oppositely charged or uncharged and immiscible with the first phase. In other embodiments, the medium includes a mixture of active molecules and charged particles. The microcells include an opening, and the opening is spanned by a porous diffusion layer, thus the porous diffusion layer can be blocked with the charged particles, the rate at which the active ingredient is dispensed can be controlled. Where the medium includes a charged first phase, the first phase can be moved adjacent to, or away from, the porous diffusion layer in order to control the rate of delivery. The microcell arrays may be loaded with different active ingredients, thereby providing a mechanism to deliver different or complimentary active ingredients on demand.

In addition to more conventional applications, such as transdermal delivery of pharmaceutical compounds, the active molecule delivery system may be the basis for delivering agricultural nutrients. The microcell arrays can be fabricated in large sheets that can be used in conjunction with hydroponic growing systems, or they can be integrated into hydrogel film farming, such as demonstrated by Mebiol, Inc. (Kanagawa, Japan). The active molecule delivery systems can be incorporated into the structural walls of smart packing, as well. The delivery system, for example, makes it possible to have long term release of antioxidants into a package containing fresh vegetables. Such packaging will dramatically improve the shelf life of certain foods, yet will only require the amount of antioxidant necessary to maintain freshness until the package is opened.

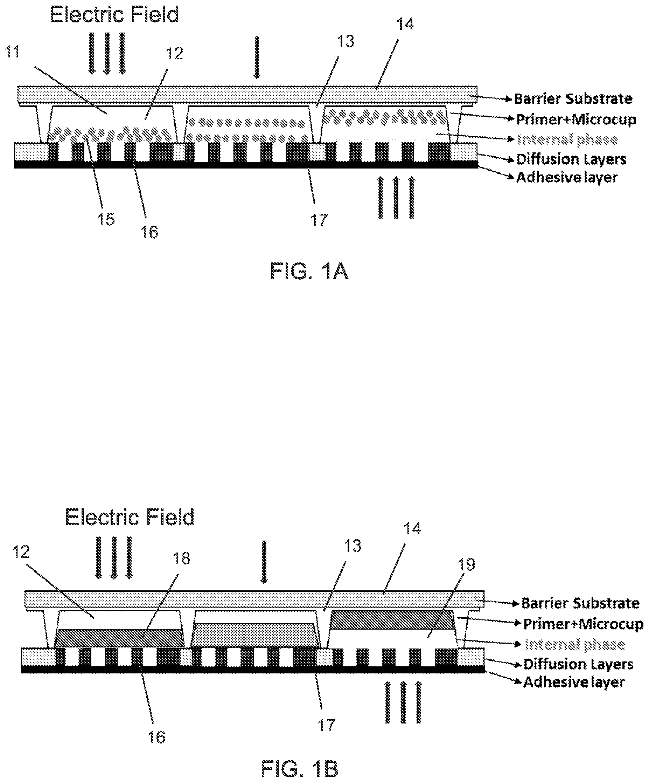

An overview of an active molecule delivery system is shown in FIG. 1A. The system includes a plurality of microcells 11, each microcell including a medium 12 (a.k.a. internal phase), that includes an active molecule and a charged particle 15. Each microcell 11 is part of an array that is formed from a polymer matrix 13, which is described in more detail below. The active molecule delivery system will typically include a backing barrier 14 to provide structural support and protection against moisture ingress and physical interactions. A portion of the microcell 11 will have an opening that is spanned by a porous diffusion layer 16, which may be constructed from a variety of natural or non-natural polymers, such as comprises acrylates, methacrylates, polycarbonates, polyvinyl alcohols, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polypropylene, polybutylene, polyisobutylene, hydroxymethylcellulose, or polystyrene. Often the system will additionally include an adhesive layer 17 that is also porous to the active molecule. The adhesive layer 17 assists in keeping the active molecule delivery system adjacent to the surface. As shown in FIG. 1A, the particles 15 can be caused to move within the microcell 11 such that the particles 15 restrict the motion of the active molecules through the porous diffusion layer 16.

The charged particles will typically comprise a charged core with a surrounding polymer layer. Methods for constructing such particles can be found, for example, in U.S. Patent Publication No. 2015/0301425. Thus, the core particle may be an inorganic or an organic material, such as TiO₂, BaSO₄, ZnO, metal oxides, manganese ferrite black spinel, copper chromite black spinel, carbon black or zinc sulfide pigment particles. In some embodiments, the charged particles may have a surface treatment that increases the charge density on the core particle. For example, the core particle may be surface treated with an organic silane having functional groups, such as acrylate, vinyl, -NH₂, -NCO, -OH or the like, for example a polyacrylate, polyurethane, polyurea, polyethylene, polyester, polysiloxane or the like. For example, a polyacrylate shell may be formed from monomer, such as styrene, methyl acrylate, methyl methacrylate, n-butyl acrylate, n-butyl methacrylate, t-butyl acrylate, t-butyl methacrylate, vinyl pyridine, n-vinyl pyrrolidone, 2-hydoxyethyl acrylate, 2-hydroxyethyl methacrylate, dimethylaminoethyl methacrylate or the like. A polyurethane shell may be formed from monomer or oligomer, such as multifunctional isocyanate or thioisocyanate, primary alcohol or the like. A polyurea shell may also be formed from monomer containing reactive groups, such as amine/isocyanate, amine/thioisocyanate or the like. The charged particles are additionally covered with steric-stabilizing polymers. The steric stabilizing polymers may be covalently bound to the surface of the charged core particles or the steric stabilizers may be merely associated with the core particle. Such stabilizing molecules usually formed of high molecular weight polymers, such as polyethylene, polypropylene, polyester, polysiloxane or a mixture thereof. The steric stabilizers should be compatible with the solvent in which the composite pigment particles are dispersed to facilitate dispersion of the composite pigment particles in the solvent. Methods for preparing such charged particle are described in U.S. Patent Publication No. 2015/0301425.

In other embodiments, the medium 12 includes a mixture of a first phase 18, which is charged and includes the active molecules, and a second phase 19, which is oppositely charged or uncharged, but immiscible with the first phase. With the application of an electric field, the charged first phase is driven adjacent to the porous diffusion layer, thereby allowing the active molecules to transit the porous diffusion layer 16 (see leftmost microcell). However, when the polarity of the electric field is reversed, the first phase 18 will be driven away from the porous diffusion layer 16 and prevent the active from traversing the porous diffusion layer 16 (see rightmost microcell). In some embodiments, there will be an equal volume of the first and second phases in each microcell. In other embodiments, there may be a smaller volume of the charged first phase than the second phase, for example there may be a 1:2 ratio, or a 1:4 ratio, or a 1:10 ratio of first to second phases. In other embodiments, there may be a greater volume of the charged first phase than the second phase, for example there may be a 2:1 ratio, or a 4:1 ratio, or a 10:1 ratio of first to second phases.

As described above, the mixture of phases will typically include one hydrophilic and one hydrophobic component. The phase in which the active is located will be dependent upon the solubility of the active. Typically, the active will be in a charged phase, thereby providing greater control of delivery, however it is possible that a charged layer that does not include the active can be used to gate the active's access to the porous diffusion layer. Accordingly, the first phase first charged phase may comprise a biocompatible non-polar liquid or an aqueous liquid. If the first phase is a biocompatible non-polar liquid, that liquid could be a vegetable, fruit, or nut oil. The biocompatible non-polar liquid may additionally include charged hydrophobic polymers, such as functionalized acrylates, for example N-methacryloyl-L-phenyl alanine (MAPA). In other embodiments, the first charged phase includes an aqueous liquid. The aqueous liquid may also include charged hydrophilic polymers, such as polyethylenimine (PEI), poly(ethylene glycol) (PEG), poly(vinyl alcohol) (PVA), poly(vinylpyrrolidone) (PVP) or copolymers of any of these polymers. In some embodiments, the first charged phase includes a phosphate buffer. Whether the active and the first charged phase is hydrophilic or hydrophobic will depend in good part upon the nature of the active to be delivered, because the active will typically be in the first phase. Accordingly, where the first charged phase is hydrophilic the second phase will be hydrophobic, and where the first charged phase is hydrophobic the second phase will be hydrophilic. The second phase may include any of the components above, as needed to engineer the proper response for the first charged phase. In some embodiments, the second phase will be charged, however, in other embodiments, the second phase will not be charged.

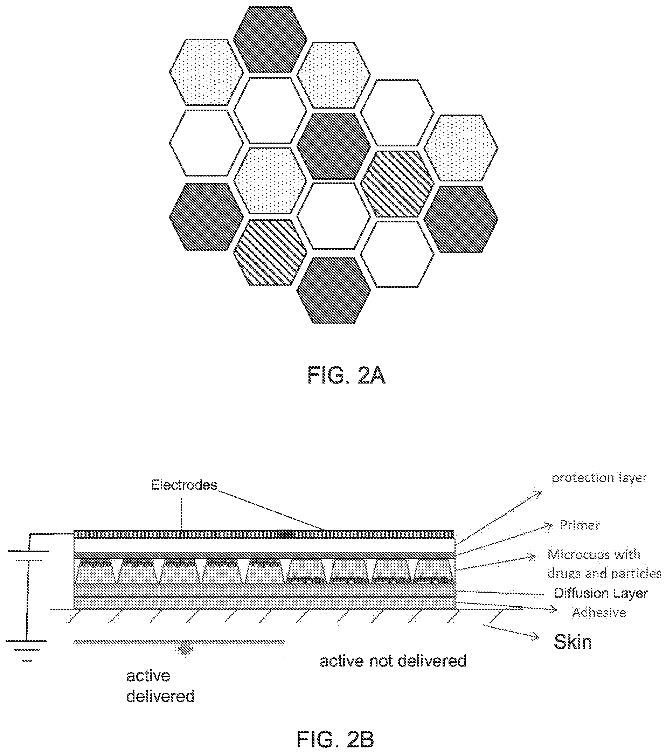

In addition to regulating the flow of active molecules, the microcell construction of the invention lends itself to making arrays of differing active molecules, or arrays of different concentrations, as illustrated in FIG. 2A. Using picoliter injection with inkjet or other fluidic systems, individual microcells can be filled to enable a variety of different actives to be included in an active molecule delivery system. For example, a system of the invention may include nicotine at four different concentrations, thereby allowing different dosages to be delivered at different times during the day. For example, shortly after waking the most concentrated dose may be delivered (dark gray), followed by a much lower taper dose during the day (speckled), until the time that a user needs another more concentrated dose. It is possible to also include different actives in the same microcell. For example, the system of FIG. 2A may also include an analgesic (stripes) to reduce swelling and itching in the area of the skin that is in contact with the delivery system. Of course, a variety of combinations are possible, and varying microcells might include pharmaceuticals, nutraceuticals, adjuvants, vitamins, or vaccines. Furthermore, the arrangement of the microcells may not be distributed. Rather the microcells may be filled in clusters, which makes filling and driving more straightforward. In other embodiments, smaller microcell arrays may be filled with the same medium, i.e., having the same active molecule at the same concentration, and then the smaller arrays assembled into a larger array to make a delivery system of the invention.

As shown in FIG. 2B, this distributed arrangement also allows for fine control of the amount of active delivered. For example five microcells can be "open" while four microcells are "closed." Regarding FIG. 2B, with the application of a suitable electric field, the charged particles in the left-hand microcells move away from the diffusion layer, allowing the actives molecules to be delivered while on the right-hand side particles blocking passage of actives across the diffusion layer. Of course these same ideas may be used to deliver multiple different actives at the same or different times. This differential rate limiting can be achieved by activating electrodes over the portion of the microcells for which passage is to be slowed. While a mixed phase system, similar to FIG. 1B, is not shown in FIG. 2B, it is understood that the same principles of localized electric field driving can be used to control delivery from the two phase system.

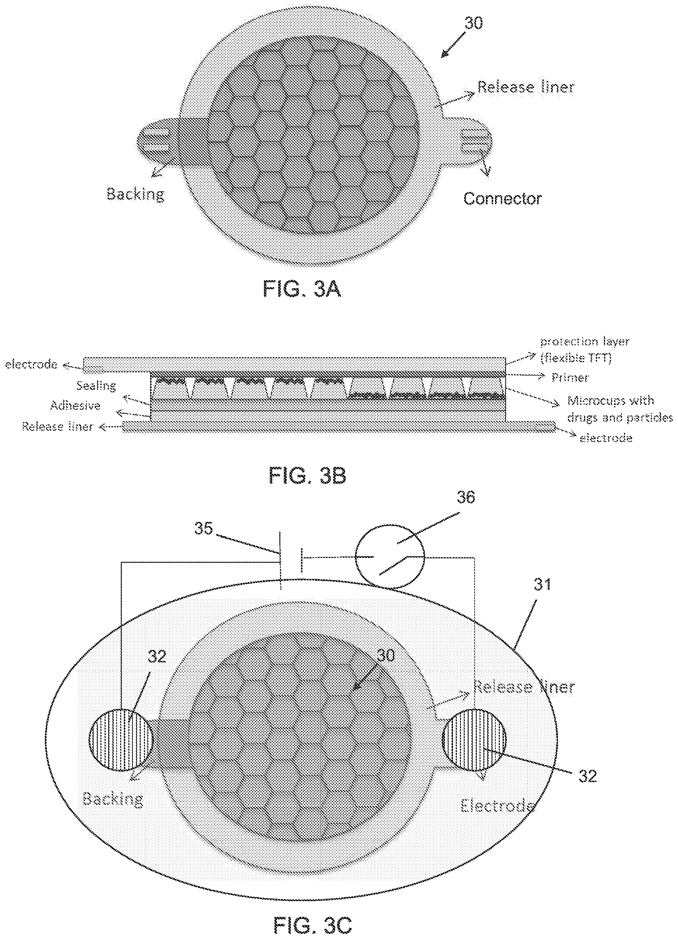

A top view of an embodiment of an active molecule delivery system of the invention is shown in FIG. 3A and a side view is shown in FIG. 3B. The electrodes may be configured to be of varying areas (as shown) or the electrodes may be of approximately the same area. In many embodiments, the electrodes will include connectors to allow the electrodes to be interfaced to a voltage source. The embodiment of FIG. 3A and 3B actually includes a flexible thin film transistor array (TFT) that allows for individual microcells to be biased in order to control delivery from the individual microcells. An active molecule delivery system of the invention may also include a backing layer and/or a protective covering (see FIG. 3C). In addition to the adhesive layer, the active molecule delivery system may also include a release liner that assures that the active is not exposed to air or other contaminants during storage. When using this active matrix delivery system, a user will apply the system and connect to the electrodes to a voltage source, whereupon the voltage source activates the patch by applying the selected waveform to the patch.

A controller for an active molecule delivery system of the invention is shown in FIG. 3C. The controller includes an active molecule delivery system 30, including a medium having actives and charged particles, or actives in a first phase of a two phase system, a voltage source 35, and a protective covering 31. The voltage source 35 is typically a battery, however the voltage source 35 may be a photovoltaic cell, or it may be a wireless power receiver. The protective covering 31 may be a rigid polymer, such as polypropylene or polyvinyl chloride, or the protective covering may be flexible such as an elastomeric film or cloth. In most embodiments the electrodes of the active molecule delivery system 30 will be coupled to the voltage source 35 via connectors 32. In some embodiments, the connectors 32 are configured to allow the active molecule delivery system 30 to be replaceable. That is, once the actives from the active molecule delivery system 30 have been administered, the active molecule delivery system 30 can be removed from the controller and a new active molecule delivery system 30 put in its place. In such embodiments, the voltage source 35 may be a rechargeable battery to allow for the controller to be used with a large number of active molecule delivery systems 30. In most embodiments, the connection between the voltage source 35 and the connectors 32 will be interruptible with a switch 36, which could be a mechanical switch, i.e., a pushbutton, or a digital switch, i.e., a transistor. The switch 36 will allow the voltage to be turned on and off with respect to various microcells to thereby meter the administration of the active. In many embodiments, the controller will additionally include a processor for controlling the switch. In some embodiments, there are a plurality of switches, for example a transistor array whereby the microcells can be addressed in a pixel-like fashion using row and column drivers.

In some embodiments, the controller has a size and shape similar to a handheld electrical shaver. The controller may have only a few features or the controller may have a variety of functionality. For example, the controller may include a dial with either numbers such as 1 to 9 or with different usage modes can be embedded in the applicator and the user can choose different amount of drugs or different duration the user plans to use the patch by adjusting the dial before activate the patch. It's also possible to adjust based on the user's age or skin condition by setting up different levels on the controller. The numbers or various user modes on the dial correspond to various waveforms so that the activation of patch can be personalized. In some embodiments, where the controller is reusable, a user can removed the controller from the skin, remove the active molecule delivery device, insert a new active molecule delivery device, remove the release liner of the newly loaded device and replace on the skin.

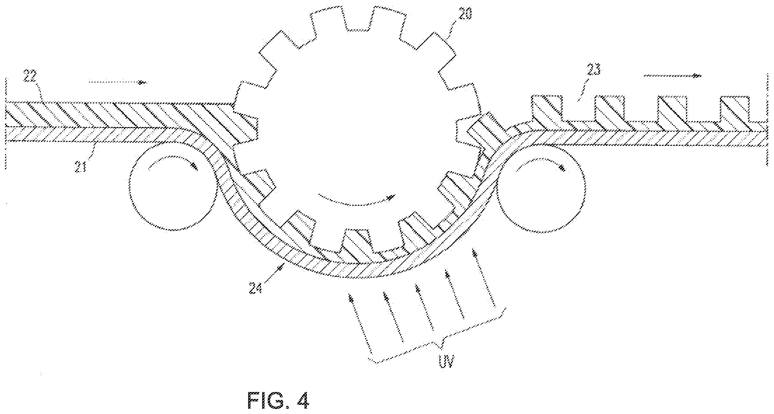

**Techniques for constructing microcells.** Microcells may be formed either in a batchwise process or in a continuous roll-to-roll process as disclosed in U.S. Pat. No. 6,933,098. The latter offers a continuous, low cost, high throughput manufacturing technology for production of compartments for use in a variety of applications including active molecule delivery and electrophoretic displays. Microcell arrays suitable for use with the invention can be created with microembossing, as illustrated in FIG. 4. A male mold 20 may be placed either above the web 24, as shown in FIG.4, or below the web 24 (not shown) however alternative arrangements are possible. See U.S. Patent No. 7,715,088. A conductive substrate may be constructed by forming a conductor film 21 on polymer substrate that becomes the backing for a device. A composition comprising a thermoplastic, thermoset, or a precursor thereof 22 is then coated on the conductor film. The thermoplastic or thermoset precursor layer is embossed at a temperature higher than the glass transition temperature of the thermoplastics or thermoset precursor layer by the male mold in the form of a roller, plate or belt.

The thermoplastic or thermoset precursor for the preparation of the microcells may be multifunctional acrylate or methacrylate, vinyl ether, epoxide and oligomers or polymers thereof, and the like. A combination of multifunctional epoxide and multifunctional acrylate is also very useful to achieve desirable physico-mechanical properties. A crosslinkable oligomer imparting flexibility, such as urethane acrylate or polyester acrylate, may be added to improve the flexure resistance of the embossed microcells. The composition may contain polymer, oligomer, monomer and additives or only oligomer, monomer and additives. The glass transition temperatures (or T_{g}) for this class of materials usually range from about -70° C. to about 150° C., preferably from about -20° C. to about 50° C. The microembossing process is typically carried out at a temperature higher than the T_{g}. A heated male mold or a heated housing substrate against which the mold presses may be used to control the microembossing temperature and pressure.

As shown in FIG. 4, the mold is released during or after the precursor layer is hardened to reveal an array of microcells 23. The hardening of the precursor layer may be accomplished by cooling, solvent evaporation, cross-linking by radiation, heat or moisture. If the curing of the thermoset precursor is accomplished by UV radiation, UV may radiate onto the transparent conductor film from the bottom or the top of the web as shown in the two figures. Alternatively, UV lamps may be placed inside the mold. In this case, the mold must be transparent to allow the UV light to radiate through the pre-patterned male mold on to the thermoset precursor layer. A male mold may be prepared by any appropriate method, such as a diamond turn process or a photoresist process followed by either etching or electroplating. A master template for the male mold may be manufactured by any appropriate method, such as electroplating. With electroplating, a glass base is sputtered with a thin layer (typically 3000 Å) of a seed metal such as chrome inconel. The mold is then coated with a layer of photoresist and exposed to UV. A mask is placed between the UV and the layer of photoresist. The exposed areas of the photoresist become hardened. The unexposed areas are then removed by washing them with an appropriate solvent. The remaining hardened photoresist is dried and sputtered again with a thin layer of seed metal. The master is then ready for electroforming. A typical material used for electroforming is nickel cobalt. Alternatively, the master can be made of nickel by electroforming or electroless nickel deposition. The floor of the mold is typically between about 50 to 400 microns. The master can also be made using other microengineering techniques including e-beam writing, dry etching, chemical etching, laser writing or laser interference as described in "Replication techniques for micro-optics", SPIE Proc. Vol. 3099, pp. 76-82 (1997). Alternatively, the mold can be made by photomachining using plastics, ceramics or metals.

Prior to applying a UV curable resin composition, the mold may be treated with a mold release to aid in the demolding process. The UV curable resin may be degassed prior to dispensing and may optionally contain a solvent. The solvent, if present, readily evaporates. The UV curable resin is dispensed by any appropriate means such as, coating, dipping, pouring or the like, over the male mold. The dispenser may be moving or stationary. A conductor film is overlaid the UV curable resin. Pressure may be applied, if necessary, to ensure proper bonding between the resin and the plastic and to control the thickness of the floor of the microcells. The pressure may be applied using a laminating roller, vacuum molding, press device or any other like means. If the male mold is metallic and opaque, the plastic substrate is typically transparent to the actinic radiation used to cure the resin. Conversely, the male mold can be transparent and the plastic substrate can be opaque to the actinic radiation. To obtain good transfer of the molded features onto the transfer sheet, the conductor film needs to have good adhesion to the UV curable resin which should have a good release property against the mold surface.

Microcell arrays for the invention typically include a pre-formed conductor film, such as indium tin oxide (ITO) conductor lines, however other conductive materials, such as silver or aluminum may be used. The conductive material may be backed by or integrated into substrates such as polyethylene terephthalate, polyethylene naphthalate, polyaramid, polyimide, polycycloolefin, polysulfone, epoxy and their composites. The conductor film may coated with a radiation curable polymer precursor layer. The film and precursor layer are then exposed imagewise to radiation to form the microcell wall structure. Following exposure, the precursor material is removed from the unexposed areas, leaving the cured microcell walls bonded to the conductor film/support web. The imagewise exposure may be accomplished by UV or other forms of radiation through a photomask to produce an image or predetermined pattern of exposure of the radiation curable material coated on the conductor film. Although it is generally not required, the mask may be positioned and aligned with respect to the conductor film, i.e., ITO lines, so that the transparent mask portions align with the spaces between ITO lines, and the opaque mask portions align with the ITO material (intended for microcell cell floor areas).

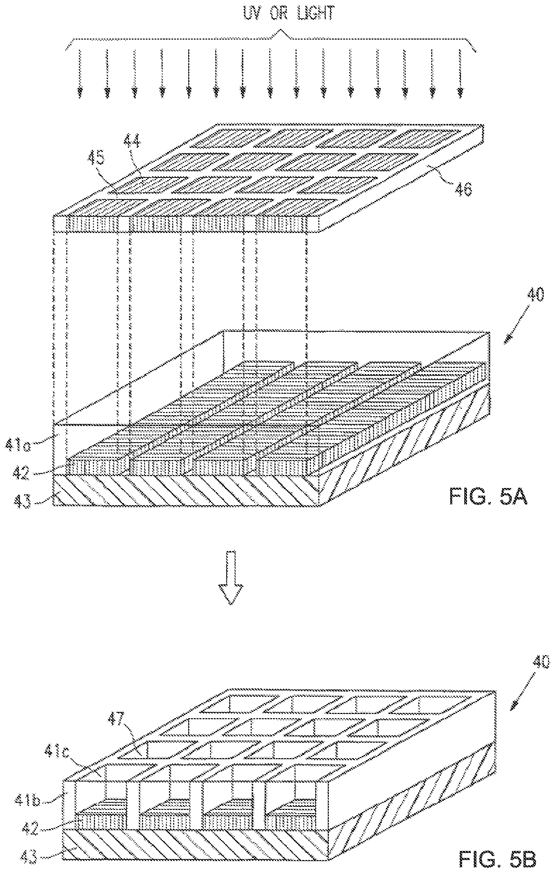

**Photolithography.** Microcells can also be produced using photolithography. Photolithographic processes for fabricating a microcell array are illustrated in FIGS. 5A and 5B. As shown in FIGS. 5A and 5B, the microcell array 40 may be prepared by exposure of a radiation curable material 41a coated by known methods onto a conductor electrode film 42 to UV light (or alternatively other forms of radiation, electron beams and the like) through a mask 46 to form walls 41b corresponding to the image projected through the mask 46. The base conductor film 42 is preferably mounted on a supportive substrate base web 43, which may comprise a plastic material.

In the photomask 46 in FIG. 5A, the dark squares 44 represent the opaque area and the space between the dark squares represents the transparent area 45 of the mask 46. The UV radiates through the transparent area 45 onto the radiation curable material 41a. The exposure is preferably performed directly onto the radiation curable material 41a, i.e., the UV does not pass through the substrate 43 or base conductor 42 (top exposure). For this reason, neither the substrate 43, nor the conductor 42, needs to be transparent to the UV or other radiation wavelengths employed.

As shown in FIG. 5B, the exposed areas 41b become hardened and the unexposed areas (protected by the opaque area 44 of the mask 46) are then removed by an appropriate solvent or developer to form the microcells 47. The solvent or developer is selected from those commonly used for dissolving or reducing the viscosity of radiation curable materials such as methylethylketone (MEK), toluene, acetone, isopropanol or the like. The preparation of the microcells may be similarly accomplished by placing a photomask underneath the conductor film/substrate support web and in this case the UV light radiates through the photomask from the bottom and the substrate needs to be transparent to radiation.

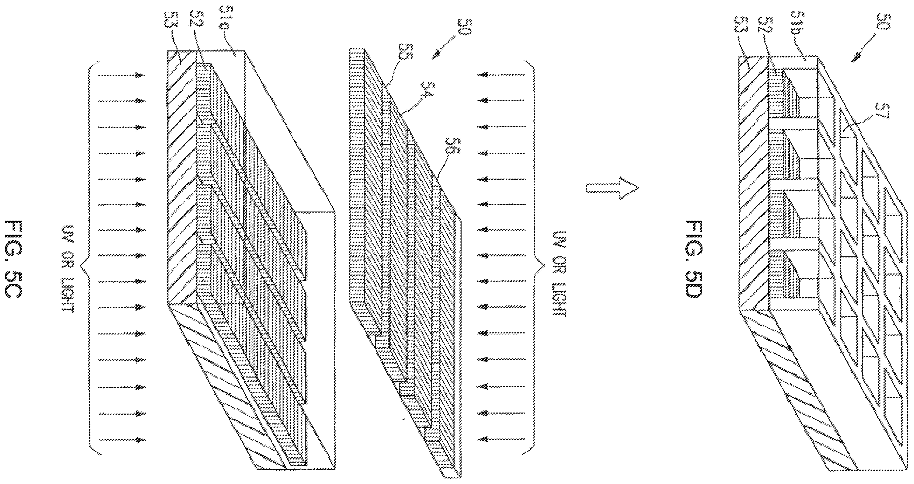

**Imagewise Exposure.** Still another alternative method for the preparation of the microcell array of the invention by imagewise exposure is illustrated in FIGS. 5C and 5D. When opaque conductor lines are used, the conductor lines can be used as the photomask for the exposure from the bottom. Durable microcell walls are formed by additional exposure from the top through a second photomask having opaque lines perpendicular to the conductor lines. FIG. 5C illustrates the use of both the top and bottom exposure principles to produce the microcell array 50 of the invention. The base conductor film 52 is opaque and line-patterned. The radiation curable material 51a, which is coated on the base conductor 52 and substrate 53, is exposed from the bottom through the conductor line pattern 52 which serves as the first photomask. A second exposure is performed from the "top" side through the second photomask 56 having a line pattern perpendicular to the conductor lines 52. The spaces 55 between the lines 54 are substantially transparent to the UV light. In this process, the wall material 51b is cured from the bottom up in one lateral orientation, and cured from the top down in the perpendicular direction, joining to form an integral microcell 57. As shown in FIG. 5D, the unexposed area is then removed by a solvent or developer as described above to reveal the microcells 57. The technique described in FIGS. 5C and 5D thus allow the different walls to be constructed with different porosity, as needed for the embodiment illustrated in FIG. 2.

The microcells may be constructed from thermoplastic elastomers, which have good compatibility with the microcells and do not interact with the electrophoretic media. Examples of useful thermoplastic elastomers include ABA, and (AB)n type of di-block, tri-block, and multi-block copolymers wherein A is styrene, α-methylstyrene, ethylene, propylene or norbonene; B is butadiene, isoprene, ethylene, propylene, butylene, dimethylsiloxane or propylene sulfide; and A and B cannot be the same in the formula. The number, n, is ≧1, preferably 1-10. Particularly useful are di-block or tri-block copolymers of styrene or ox-methylstyrene such as SB (poly(styrene-b-butadiene)), SBS (poly(styrene-b-butadiene-b-styrene)), SIS (poly(styrene-b-isoprene-b-styrene)), SEBS (poly(styrene-b-ethylene/butylenes-b-stylene)) poly(styrene-b-dimethylsiloxane-b-styrene), poly((α-methylstyrene-b-isoprene), poly(α-methylstyrene-b-isoprene-b-α-methylstyrene), poly(α-methylstyrene-b-propylene sulfide-b-α-methyl styrene), poly(α-methylstyrene-b-dimethylsiloxane-b-α-methylstyrene). Commercially available styrene block copolymers such as Kraton D and G series (from Kraton Polymer, Houston, Tex.) are particularly useful. Crystalline rubbers such as poly(ethylene-co-propylene-co-5-methylene-2-norbomene) or EPDM (ethylene-propylene-diene terpolymer) rubbers such as Vistalon 6505 (from Exxon Mobil, Houston, Tex.) and their grafted copolymers have also been found very useful.

The thermoplastic elastomers may be dissolved in a solvent or solvent mixture which is immiscible with the display fluid in the microcells and exhibits a specific gravity less than that of the display fluid. Low surface tension solvents are preferred for the overcoating composition because of their better wetting properties over the microcell walls and the electrophoretic fluid. Solvents or solvent mixtures having a surface tension lower than 35 dyne/cm are preferred. A surface tension of lower than 30 dyne/cm is more preferred. Suitable solvents include alkanes (preferably C₆₋₁₂ alkanes such as heptane, octane or Isopar solvents from Exxon Chemical Company, nonane, decane and their isomers), cycloalkanes (preferably C₆₋₁₂ cycloalkanes such as cyclohexane and decalin and the like), alkylbezenes (preferably mono- or di-C₁₋₆ alkyl benzenes such as toluene, xylene and the like), alkyl esters (preferably C₂₋₅ alkyl esters such as ethyl acetate, isobutyl acetate and the like) and C₃₋₅ alkyl alcohols (such as isopropanol and the like and their isomers). Mixtures of alkylbenzene and alkane are particularly useful.

In addition to polymer additives, the polymer mixtures may also include wetting agents (surfactants). Wetting agents (such as the FC surfactants from 3M Company, Zonyl fluorosurfactants from DuPont, fluoroacrylates, fluoromethacrylates, fluoro-substituted long chain alcohols, perfluoro-substituted long chain carboxylic acids and their derivatives, and Silwet silicone surfactants from OSi, Greenwich, Conn.) may also be included in the composition to improve the adhesion of the sealant to the microcells and provide a more flexible coating process. Other ingredients including crosslinking agents (e.g., bisazides such as 4,4'-diazidodiphenylmethane and 2,6-di-(4'-azidobenzal)-4-methylcyclohexanone), vulcanizers (e.g., 2-benzothiazolyl disulfide and tetramethylthiuram disulfide), multifunctional monomers or oligomers (e.g., hexanediol, diacrylates, trimethylolpropane, triacrylate, divinylbenzene, diallylphthalene), thermal initiators (e.g., dilauroryl peroxide, benzoyl peroxide) and photoinitiators (e.g., isopropyl thioxanthone (ITX), Irgacure 651 and Irgacure 369 from Ciba-Geigy) are also highly useful to enhance the physico-mechanical properties of the sealing layer by crosslinking or polymerization reactions during or after the overcoating process.

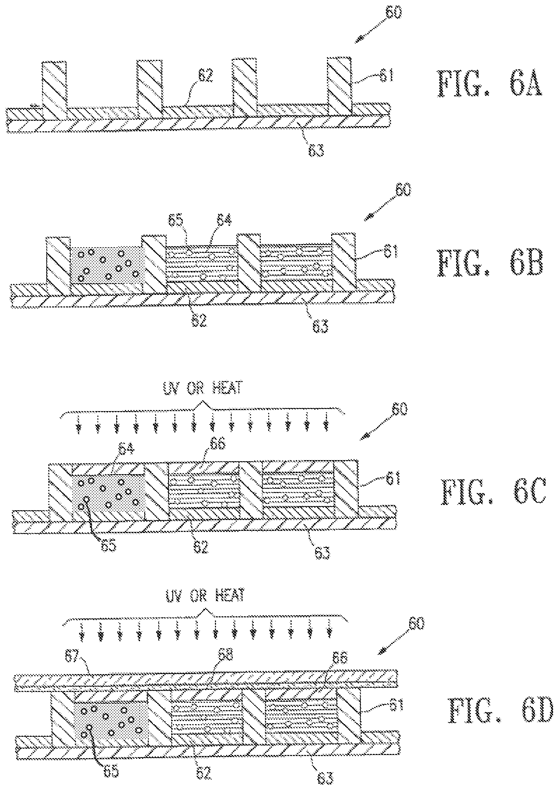

After the microcells are produced, they are filled with appropriate media, including active molecules for delivery. The media may include charged particles, or the media may comprise a two phase system. The microcell array 60 may be prepared by any of the methods described above. As shown in cross-section in FIGS. 6A-6D, the microcell walls 61 extend upward from the substrate 63 to form the open cells. Primer 62 may be added, as needed, to keep the media from interacting with the walls of the microcells. Prior to filling, the microcell array 60 may be cleaned and sterilized to assure that the active molecules are not compromised prior to use. After construction an electrode may be affixed to the filled cells or an active matrix of electrodes, e.g., to allow individual microcells to be addressed.

As shown in FIG. 6B, different microcells may include different active molecules. In other embodiments the different microcells may include differing types of charged particles or the different microcells may include magnetic particles. The microcells 60 are preferably partially filled to prevent overflow and the unintentional mixing of active ingredients. In systems for delivering hydrophobic active molecules, the mixture may be based upon a biocompatible oil or some other biocompatible hydrophobic carrier. For example, the mixture may comprise a vegetable, fruit, or nut oil. In other embodiments, silicone oils may be used. In systems for delivering hydrophilic active molecules, the mixture may be based upon water or another aqueous medium such as phosphate buffer. The mixture need not be a liquid, however, as hydrogels and other matrices may be suitable to deliver the active ingredients provided that the structure of the matrix does not restrict motion of the charged or magnetic particles to the point that they cannot move to the porous diffusion layer to limit the delivery of the active molecules.

The microcells may be filled using a variety of techniques. In some embodiments, where a large number of neighboring microcells are to be filled with an identical mixture, blade coating may be used to fill the microcells to the depth of the microcell walls 61. In other embodiments, where a variety of different mixtures are to be filled in a variety of nearby microcell, inkjet-type microinjection can be used to fill the microcells. In yet other embodiments, microneedle arrays may be used to fill an array of microcells with the correct mixtures. The filling may be done in a one-step or multistep process. For example, all of the cells may be partially filled with an amount of solvent and as well as charged or magnetic particles. The partially filled microcells are then filled with a second mixture including the solvent and one or more active molecules to be delivered.

As shown in FIG. 6C, after filling, the microcells are sealed by applying a polymer 66 that becomes the porous diffusion layer. In some embodiments, the sealing process may involve exposure to heat, dry hot air, or UV radiation. In most embodiments the polymer 66 will be compatible with the mixture 64, but not dissolved by the solvent of the mixture 64. The polymer 66 will also be biocompatible and selected to adhere to the sides or tops of the microcell walls 61. A suitable biocompatible adhesive for the porous diffusion layer is a phenethylamine mixture, such as described in U.S. Patent Application No. 15/336,841, filed October 30, 2016 and titled "Method for Sealing Microcell Containers with Phenethylamine Mixtures," which is incorporated herein by reference in its entirety. Accordingly, the final microcell structure is mostly impervious to leaks and able to withstand flexing without delamination of the porous diffusion layer.

In alternate embodiments, a variety of individual microcells may be filled with the desired mixture by using iterative photolithography. The process typically includes coating an array of empty microcells with a layer of positively working photoresist, selectively opening a certain number of the microcells by imagewise exposing the positive photoresist, followed by developing the photoresist, filling the opened microcells with the desired mixture, and sealing the filled microcells by a sealing process. These steps may be repeated to create sealed microcells filled with other mixtures. This procedure allows for the formation of large sheets of microcells having the desired ratio of mixtures or concentrations.

After the microcells 60 are filled, the sealed array may be laminated with a second conductive film 68 that is also porous to the active molecules, preferably by pre-coating the conductive film 68 with an adhesive layer which may be a pressure sensitive adhesive, a hot melt adhesive, or a heat, moisture, or radiation curable adhesive. The laminate adhesive may be post-cured by radiation such as UV through the top conductor film if the latter is transparent to the radiation. In other embodiments, an active matrix of electrodes may be bonded directly to the sealed array of electrophoretic microcell cells. In some embodiments, a biocompatible adhesive 67 is then laminated to the assembly. The biocompatible adhesive 67 will allow active molecules to pass through while keeping the device mobile on a user. Suitable biocompatible adhesives are available from 3M (Minneapolis, MN).

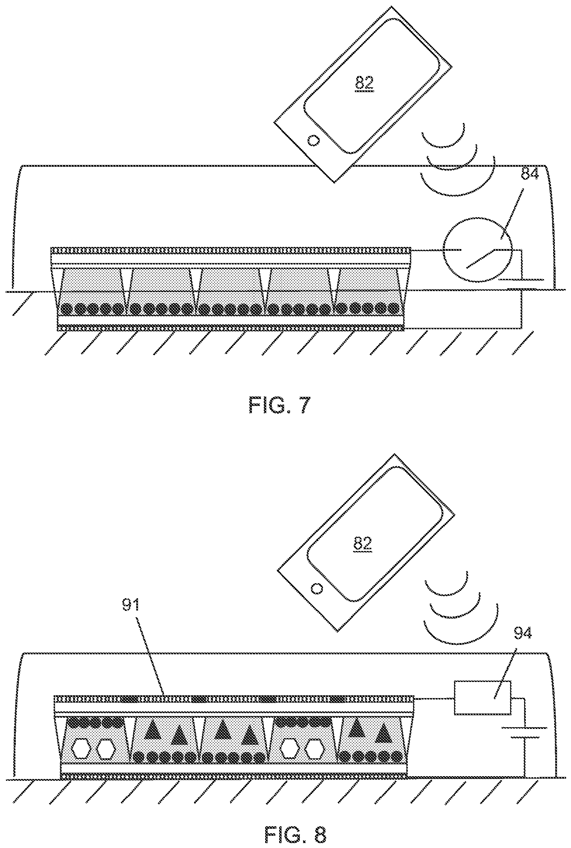

Advanced embodiments of an active molecule delivery system will include circuitry to allow the active molecule delivery system to be controlled wirelessly with a secondary device 82, such as a smart phone or smart watch. As shown in FIG. 7, a simple system will allow a user to open an electronic/digital switch 84 which will cause an electric field to be delivered to electrodes, thereby driving charged particles away from the porous diffusion layer and giving the user a dose of the active molecules. In advanced embodiments, i.e., as shown in FIG. 8, the active molecule delivery system will include an active matrix of electrodes 91 that are controlled by a controller 94, which is also able to receive wireless signals from a secondary device 82. The embodiment of FIG. 8 will allow a user to control, for example, the type of active molecule that is delivered and the amount. Using an application on the secondary device 82 it may be possible to program the active molecule delivery system to modify the amount of active molecule delivered based upon the time of day. In other embodiments, the application may be operatively connected to biometric sensors, e.g., a fitness tracker, whereby the application causes the dosage to be turned off if, e.g., the pulse rate of the user exceeds a preset threshold.

When driving the patches of FIGS. 7 and 8, NFC, Bluetooth, WIFI, or other wireless communication function is turned on allowing a user to manipulate the charged or magnetic particles in the microcells to move them to different positon electrophoretically or magnetically. The patch driving can be done before or after the patch is applied on skin surface and drug release adjustment can be achieved at any time when necessary by re-driving the patch due to the battery-free feature. When the charged or magnetic particles are driven to different positions, the releasing profiles will be altered due to interaction with the porous diffusion layer. Because the driving is controlled by smart watch or smart phone, the percentage and area for all of the microcells at different driving status is known, which means all of the usage data will be available to a provider or therapist, including when the patch is activated and what amount of active is administered. For the "on demand" function, whenever the patient or the doctor feel the necessity to adjust the drug releasing, the patch can be re-driven. For the "programmable" feature, because every microcell can be turned independently, the overall releasing of patch can be programmed by driving the charged particles to different level at various segments. Because the patch after driving is segmented, the skin irritation can also be controlled. Additionally, patient compliance is also good because the smart device that is used to activate the patch can also communicate with doctors remotely for data sharing.

The present invention is defined by the appended claims.

## Claims

1. An active molecule delivery system comprising:
a plurality of microcells (11), wherein each microcell includes an opening and contains
a mixture of an active molecule dispersed in a first charged phase, and
a second phase that is oppositely charged or uncharged and immiscible with the first phase;
a porous diffusion layer (16) spanning the opening of each microcell (11); and
a source of an electric field,
wherein the first charged phase comprises a biocompatible non-polar liquid or an aqueous liquid;
wherein one of the first charged phase and second phase is hydrophilic and the other phase is hydrophobic;
wherein the active molecule delivery system is configured to move the first charged phase adjacent or away from the porous diffusion layer (16) by application of the electric field.

2. The active molecule delivery system of claim 1, wherein the biocompatible non-polar liquid is vegetable, fruit, or nut oil.

3. The active molecule delivery system of claim 1, wherein the biocompatible non-polar liquid further comprises charged hydrophobic polymers.

4. The active molecule delivery system of claim 1, wherein the aqueous liquid further comprises charged hydrophilic polymers.

5. The active molecule delivery system of claim 1, wherein the aqueous liquid is a phosphate buffer.

6. The active molecule delivery system of claim 1, further comprising an adhesive layer (17) adjacent the porous diffusion layer (16).

7. The active molecule delivery system of claim 1, wherein the source of an electric field comprises first and second electrodes and the mixture of the active molecule dispersed in the first charged phase and the second phase is sandwiched between the first and second electrodes.

8. The active molecule delivery system of claim 7, wherein the first electrode or the second electrode is part of an active matrix of electrodes and/or is porous.

9. The active molecule delivery system of claim 7, wherein the first electrode and the second electrode include connectors (32) for coupling to a portable energy supply (35).

10. The active molecule delivery system of claim 1, wherein the porous diffusion layer (16) comprises an acrylate, a methacrylate, a polycarbonate, a polyvinyl alcohol, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polypropylene, or polystyrene.

11. The active molecule delivery system of claim 1, wherein the active molecule is a pharmaceutical compound.

12. The active molecule delivery system of claim 1, wherein the plurality of microcells (11) comprises first microcells containing a mixture of first active molecules and second microcells containing (i) a mixture of second active molecules; (ii) active molecules at a different concentration from the first microcells; or (iii) an adjuvant.

13. The active molecule delivery system of claim 1, wherein each of the plurality of microcells (11) has a volume greater than 100 nL.

14. The active molecule delivery system of claim 1, wherein the porous diffusion layer (16) has an average pore size of between 10 nm and 100 µm.

15. An active molecule delivery system controller comprising:
an active molecule delivery system including:
a plurality of microcells, wherein each microcell includes an opening and contains a mixture of an active molecule and charged particles,
a porous diffusion layer spanning the opening of each microcell, and
first and second electrodes sandwiching the plurality of microcells and configured to apply an electric field;
a voltage source coupled to the first and second electrodes; and
a protective covering;
wherein the active molecule delivery system is configured to move the charged particles adjacent or away from the porous diffusion layer by application of the electric field.

16. The active molecule delivery system controller of claim 15, further comprising at least one of:
(i) a wireless receiver and a wireless transmitter;
(ii) connectors configured to couple with the first and second electrodes of the active molecule delivery system; or
(iii) a switch configured to interrupt flow from the voltage source to the active molecule delivery system.

## Patentansprüche

1. Wirkstoffmolekülzuführungssystem, umfassend:
Vielzahl von Mikrozellen (11), wobei jede Mikrozelle eine Öffnung einschließt und enthält
eine Wirkstoffmolekülmischung, die in einer ersten geladenen Phase dispergiert ist und
eine zweite Phase, die gegensätzlich geladen oder nicht geladen und unvermischbar mit der ersten Phase ist;
eine poröse Diffusionsschicht (16), die sich über die Öffnung jeder Mikrozelle (11) erstreckt; und
eine Quelle eines elektrischen Feldes,
wobei die erste geladene Phase eine biokompatible nicht polare Flüssigkeit oder eine wässrige Flüssigkeit umfasst;
wobei entweder die erste geladene oder zweite Phase hydrophil ist und die entsprechend andere Phase hydrophob ist;
wobei das Wirkstoffmolekülzuführungssystem so konfiguriert ist, dass es die erste geladene Phase entlang oder weg von der porösen Diffusionsschicht (16) durch die Anwendung eines elektrischen Feldes bewegt.

2. Wirkstoffmolekülzuführungssystem nach Anspruch 1, wobei es sich bei der biokompatiblen nicht polaren Flüssigkeit um Pflanzen-, Obst- oder Nussöl handelt.

3. Wirkstoffmolekülzuführungssystem nach Anspruch 1, wobei die biokompatible nicht polare Flüssigkeit weiter geladene hydrophobe Polymere umfasst.

4. Wirkstoffmolekülzuführungssystem nach Anspruch 1, wobei die wässrige Flüssigkeit weiter geladene hydrophile Polymere umfasst.

5. Wirkstoffmolekülzuführungssystem nach Anspruch 1, wobei es sich bei der wässrigen Flüssigkeit um einen Phosphatpuffer handelt.

6. Wirkstoffmolekülzuführungssystem nach Anspruch 1, weiter umfassend eine Haftschicht (17) angrenzend an die poröse Diffusionsschicht (16).

7. Wirkstoffmolekülzuführungssystem nach Anspruch 1, wobei die Quelle eines elektrischen Feldes erste und zweite Elektroden umfasst und die Wirkstoffmolekülmischung, die in der ersten geladenen Phase und der zweiten Phase dispergiert ist, zwischen der ersten und zweiten Elektrode befestigt ist.

8. Wirkstoffmolekülzuführungssystem nach Anspruch 7, wobei die erste Elektrode oder die zweite Elektrode Teil einer aktiven Matrix an Elektroden und/oder porös ist.

9. Wirkstoffmolekülzuführungssystem nach Anspruch 7, wobei die erste Elektrode und die zweite Elektrode Anchlüsse (32) zum Koppeln an eine tragbare Energieversorgung (35) einschließen.

10. Wirkstoffmolekülzuführungssystem nach Anspruch 1, wobei die poröse Diffusionsschicht (16) ein Acrylat, ein Methacrylat, ein Polycarbonat, einen Polyvinylalkohol, Cellulose, Poly(N-isopropylacrylamid) (PNIPAAm), Poly(milchsäure-co-glykolsäure) (PLGA), Polyvinylidenchlorid, Acrylnitril, amorphes Nylon, orientierten Polyester, Terephthalat, Polyvinylchlorid, Polyethylen, Polypropylen oder Polystyrol umfasst.

11. Wirkstoffmolekülzuführungssystem nach Anspruch 1, wobei es sich bei dem Wirkstoffmolekül um eine pharmazeutische Verbindung handelt.

12. Wirkstoffmolekülzuführungssystem nach Anspruch 1, wobei die Vielzahl an Mikrozellen (11) erste Mikrozellen, die eine Mischung an ersten Wirkstoffmolekülen und zweiten Mikrozellen, die (i) eine Mischung an zweiten Wirkstoffmolekülen enthalten; (ii) Wirkstoffmoleküle in einer Konzentration enthalten, die unterschiedlich zu den ersten Mikrozellen ist; oder (iii) ein Hilfsmittel enthalten, enthalten.

13. Wirkstoffmolekülzuführungssystem nach Anspruch 1, wobei jede der Vielzahl an Mikrozellen (11) ein Volumen von mehr als 100 nl aufweist.

14. Wirkstoffmolekülzuführungssystem nach Anspruch 1, wobei die poröse Diffusionsschicht (16) eine durchschnittliche Porengröße zwischen 10 nm und 100 µm aufweist.

15. Wirkstoffmolekülzuführungssystemregler, umfassend: Wirkstoffmolekülzuführungssystem, einschließend:
eine Vielzahl an Mikrozellen, wobei jede Mikrozelle eine Öffnung einschließt und eine Wirkstoffmolekülmischung und geladene Partikel,
eine poröse Diffusionsschicht, die sich über die Öffnung jeder Mikrozelle erstreckt und
erste und zweite Elektroden, zwischen welchen die Vielzahl von Mikrozellen befestigt sind und die konfiguriert sind um ein elektrisches Feld zu erzeugen;
eine Spannungsquelle, die an die ersten und zweiten Elektroden gekoppelt ist; und
eine Schutzabdeckung enthält;
wobei das Wirkstoffmolekülzuführungssystem so konfiguriert ist, dass es die geladenen Partikel entlang oder weg von der porösen Diffusionsschicht durch die Anwendung eines elektrischen Feldes bewegt.

16. Wirkstoffmolekülzuführungssystemregler nach Anspruch 15, weiter umfassend wenigstens eines der Folgenden:
(i) einen kabellosen Empfänger und einen kabellosen Sender;
(ii) Anschlüsse, die konfiguriert sind um sie an die ersten und zweiten Elektroden des Wirkstoffmolekülzuführungssystems zu koppeln; oder
(iii) einen Schalter, der konfiguriert ist um den Fluss aus der Spannungsquelle des Wirkstoffmolekülzuführungssystems zu unterbrechen.

## Revendications

1. Système d'administration de molécule active comprenant :
une pluralité de microcellules (11), chaque microcellule comprenant une ouverture et contenant
un mélange d'une molécule active dispersée dans une première phase chargée, et
une deuxième phase qui est chargée de manière opposée ou non chargée et non miscible avec la première phase ;
une couche de diffusion poreuse (16) s'étendant au-dessus l'ouverture de chaque microcellule (11) ; et
une source d'un champ électrique,
dans lequel la première phase chargée comprend un liquide non polaire biocompatible ou un liquide aqueux ;
dans lequel l'une de la première phase chargée et de la deuxième phase est hydrophile et l'autre phase est hydrophobe ;
dans lequel le système d'administration de molécule active est configuré pour déplacer la première phase chargée de manière adjacente ou à l'écart de la couche de diffusion poreuse (16) par application du champ électrique.

2. Système d'administration de molécule active selon la revendication 1, dans lequel le liquide non polaire biocompatible est une huile végétale, de fruits ou de noix.

3. Système d'administration de molécule active selon la revendication 1, dans lequel le liquide non polaire biocompatible comprend en outre des polymères hydrophobes chargés.

4. Système d'administration de molécule active selon la revendication 1, dans lequel le liquide aqueux comprend en outre des polymères hydrophiles chargés.

5. Système d'administration de molécule active selon la revendication 1, le liquide aqueux étant un tampon phosphate.

6. Système d'administration de molécule active selon la revendication 1, comprenant en outre une couche d'adhésif (17) adjacente à la couche de diffusion poreuse (16) .

7. Système d'administration de molécule active selon la revendication 1, dans lequel la source d'un champ électrique comprend des première et deuxième électrodes et le mélange de la molécule active dispersée dans la première phase chargée et la deuxième phase est pris en sandwich entre les première et deuxième électrodes.

8. Système d'administration de molécule active selon la revendication 7, dans lequel la première électrode ou la deuxième électrode fait partie d'une matrice active d'électrodes et/ou est poreuse.

9. Système d'administration de molécule active selon la revendication 7, dans lequel la première électrode et la deuxième électrode comprennent des connecteurs (32) pour le couplage à une alimentation en énergie portable (35) .

10. Système d'administration de molécule active selon la revendication 1, la couche de diffusion poreuse (16) comprenant un acrylate, un méthacrylate, un polycarbonate, un poly(alcool vinylique), une cellulose, un poly(N-isopropylacrylamide) (PNIPAAm), un poly(acide lactique-co-glycolique) (PLGA), un poly(chlorure de vinylidène), l'acrylonitrile, un nylon amorphe, un polyester orienté, un téréphtalate, un poly(chlorure de vinyle), un polyéthylène, un polypropylène ou un polystyrène.

11. Système d'administration de molécule active selon la revendication 1, la molécule active étant un composé pharmaceutique.

12. Système d'administration de molécule active selon la revendication 1, dans lequel la pluralité de microcellules (11) comprend des premières microcellules contenant un mélange de premières molécules actives et des deuxièmes microcellules contenant (i) un mélange de deuxièmes molécules actives ; (ii) des molécules actives à une concentration différente de celle des premières microcellules ; ou (iii) un adjuvant.

13. Système d'administration de molécule active selon la revendication 1, chacune de la pluralité de microcellules (11) ayant un volume supérieur à 100 nL.

14. Système d'administration de molécule active selon la revendication 1, la couche de diffusion poreuse (16) ayant une taille moyenne de pores comprise entre 10 nm et 100 µm.

15. Dispositif de commande de système d'administration de molécule active comprenant :
un système d'administration de molécule active comprenant :
une pluralité de microcellules, chaque microcellule comprenant une ouverture et contenant un mélange d'une molécule active et de particules chargées,
une couche de diffusion poreuse s'étendant au-dessus de l'ouverture de chaque microcellule, et
une première et une deuxième électrodes prenant en sandwich la pluralité de microcellules et configurées pour appliquer un champ électrique ;
une source de tension couplée aux première et deuxième électrodes ; et
une couverture de protection ;
le système d'administration de molécule active étant configuré pour déplacer les particules chargées adjacentes ou à l'écart de la couche de diffusion poreuse par application du champ électrique.

16. Dispositif de commande de système d'administration de molécule active selon la revendication 15, comprenant en outre au moins l'un parmi :
(i) un récepteur sans fil et un émetteur sans fil ;
(ii) des connecteurs configurés pour se coupler aux première et deuxième électrodes du système d'administration de molécule active ; ou
(iii) un commutateur configuré pour interrompre l'écoulement de la source de tension vers le système d'administration de molécule active.
